(19) Europäisches Patentamt
European Patent Office
Office européen des brevets

(11) EP 2 394 651 A1

(12) **EUROPEAN PATENT APPLICATION**

(43) Date of publication:
**14.12.2011 Bulletin 2011/50**

(21) Application number: **10178109.4**

(22) Date of filing: **29.03.2006**

(51) Int Cl.:
***A61K 31/52*** (2006.01) ***A61K 31/522*** (2006.01)
***A61P 31/22*** (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.03.2005 US 666537 P**

(62) Document number(s) of the earlier application(s) in accordance with Art. 76 EPC:
**06758222.1 / 1 865 960**

(27) Previously filed application:
**29.03.2006 US 11460**

(71) Applicants:
- **Novartis AG**
  **4056 Basel (CH)**
- **Novartis Pharma GmbH**
  **1230 Wien (AT)**

(72) Inventors:
- **Sacks, Stephen**
  **deceased (CA)**
- **Billstein, Stephan**
  **Franklin Lakes, NJ 07417 (US)**

(74) Representative: **Leon, Susanna Iris**
**Novartis AG**
**Corporate Intellectual Property**
**P.O. Box**
**4002 Basel (CH)**

Remarks:
This application was filed on 21-09-2010 as a divisional application to the application mentioned under INID code 62.

(54) **Penciclovir or Famciclovir for the Treatment of Recurrent Genital Herpes With a One-Day Application**

(57) A method for the treatment of recurrent genital herpes, in mammals, including humans, which method comprises administering to the mammals in need of such treatment, an effective amount of penciclovir or famciclovir, or a pharmaceutically acceptable salt thereof for a treatment period of one day.

EP 2 394 651 A1

## Description

### Field of the Invention

This invention relates to the treatment of recurrent genital herpes with a one day application and to the use of compounds in the preparation of a medicament for use in a one-day treatment regimen of this condition.

### Background of the Invention

Famvir® (famciclovir), a prodrug of the antiviral agent penciclovir is a synthetic acyclic guanine derivative that works against herpesviruses by interfering with viral DNA synthesis during replication. EP-A-141927 (Beecham Group p.l.c.) discloses penciclovir, the compound of formula (A):

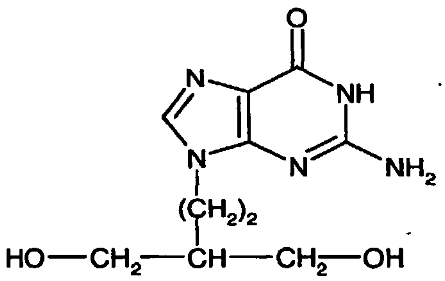

(A)

,

and salts, phosphate esters and acyl derivatives thereof, as antiviral agents. The sodium salt hydrate of penciclovir is disclosed in EP-A-216459 (Beecham Group p.l.c.). Penciclovir and its antiviral activity is also disclosed in Abstract P. V11-5, p.193 of Abstracts of 14th Int. Congress of Microbiology, Manchester, England, Sep. 7-13, 1986 (Boyd et. al.).

Orally active bioprecursors of the compound of formula (A) are of formula (B):

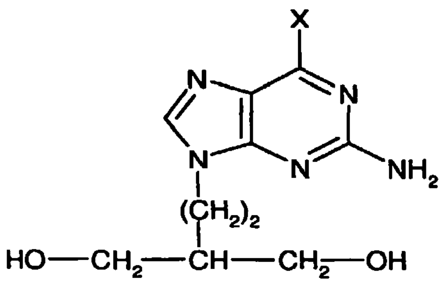

(B)

,

and salts and derivatives thereof as defined under formula (A); wherein X is $C_{1-6}$alkoxy, $NH_2$ or hydrogen. The compounds of formula (B), wherein X is $C_{1-6}$alkoxy or $NH_2$ are disclosed in EP-A-141927 and the compounds of formula (B), wherein X is hydrogen, disclosed in EP-A-182024 (Beecham Group p.l.c.) are preferred prodrugs. A particularly preferred example of a compound of formula (B) is that wherein X is hydrogen and wherein the two OH groups are in the form of the acetyl derivative, described in Example 2 of EP-A-182024, hereinafter referred to as famciclovir.

The compounds of formula (A) and (B) and salts and derivatives thereof have been described as useful in the treatment of infections caused by herpes viruses, such as herpes simplex type 1 (HSV-1), herpes simplex type 2 (HSV-2), varicella-zoster and Epstein-Barr viruses.

One in five adolescents and adults in the United States is seropositive for HSV-2, with most not aware of their infection. HSV-2 seroprevalence is higher among women than men and higher among Blacks than Whites and Mexican Americans (Smith 2002). Furthermore, infected persons shed virus even when they are asymptomatic. HSV-2 infection causes the majority of recurrent cases of genital herpes.

Genital herpes is a sexually transmitted disease. It is not usually considered life-threatening. Although it is treatable, it is not curable. After the primary infection, the virus retreats to the neural ganglia, generally those of the sacral nerve that innervate the pelvis. The virus then remains dormant until recurrence. Genital herpes may recur several times per year. Recurrences usually take less time to resolve than primary episodes and lesions heal in 6-10 days even when not treated. Furthermore, genital herpes recurs in almost all patients with a classic first infection, with over a third of persons experiencing six or more recurrences in the first year (Benedetti 1994).

Episodic or acute antiviral therapy is usually suitable for patients whose recurrences are infrequent and do not interfere greatly with the patient's lifestyle. Episodic therapy has been shown to accelerate healing, resolution of symptoms, and cessation of viral shedding. It is most successful when patients are educated to recognize prodromal symptoms and self initiate therapy as soon as they are first observed. Prompt treatment, starting within a few hours after the patient first detects symptoms of an outbreak, can also halt the process of vesicular lesion development, i.e., aborted lesions (Wald 2002).

Genital herpes has significant morbidity and impact on patients' lives. Moreover, it is a major public health problem because it strongly enhances the risk of acquiring other sexually transmitted diseases, including HIV (Corey 2004). For years, a five-day course of oral antiviral therapy was the standard. More recently, a two-day high-dose acyclovir (800 mg t.i.d.) (Wald 2002) or a 3-day valacyclovir (500 mg b.i.d.) regimen (Leone 2002), have been disclosed to be effective. However, there remains a need for therapy which alleviates or shortens the duration of symptoms associated with genital herpes. The advantages of such treatment also serves to provide an effective, convenient alternative which not only may reduce the burden on heahhcare budgets but may increase patients' compliance with treatment.

## Summary of the Invention

The present invention is to the use of Compounds of Formulae (A) and (B) as described herein, preferably famciclovir or penciclovir, for the treatment of recurrent genital herpes with a one-day treatment regimen.

## Detailed Description of the Invention

It has now been discovered that the above compounds are particularly effective in reducing duration of genital herpes lesions when given as a high-dose one-day treatment regimen. In studies performed to test efficacy of a patient-initiated one-day treatment regimen with famciclovir 1,000 mg twice a day compared to placebo in time to healing (re-epithelialization) of all non-aborted recurrent genital herpes lesions in immunocompetent patients, famciclovir reduces the median time to healing of recurrent genital herpes lesions by two days ($p<0.001$ on hazard ratio) and increases the percentage of patients with aborted lesions ($p<0.003$) as compared to placebo.

Accordingly, the present invention provides a method of treatment of recurrent genital herpes in humans, which method comprises the administration for a treatment period of one day, to a human in need of such treatment, an effective amount of a compound of formula (A):

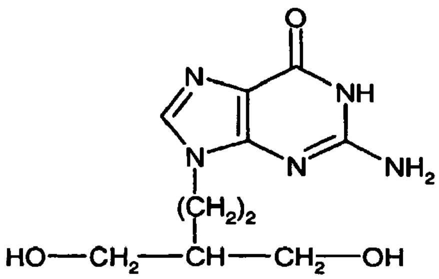

**(A)**

,

or a bioprecursor, or a pharmaceutically acceptable salt, phosphate ester and/or acyl derivative of either of the foregoing.

The term "acyl derivative" is used herein to include any derivative of the compounds of formula (A) in which one or more acyl groups are present. Such derivatives are included as bioprecursors of the compounds of formula (A) in addition to those derivatives which are *per se* biologically active.

The compound of formula (A) may be in one of the forms disclosed in EP-A-216459 (Beecham Group p.l.c.).

Examples of bioprecursors, pharmaceutically acceptable salts and derivatives are as described in the aforementioned European Patent references, the subject matter of which are incorporated herein by reference.

A particular compound of formula (B) of interest is 9-(4-acetoxy-3-acetoxymethylbut-1-yl)-2-aminopurine, known as famciclovir (FCV), the well-absorbed oral form of penciclovir (PCV).

The compound of formula (A), bioprecursors, salts and derivatives may be prepared as described in the aforementioned European Patent references.

The compound, in particular, famciclovir, may be administered by the oral route to humans and may be compounded in the form of syrup, tablets or capsule. When in the form of a tablet, any pharmaceutical carrier suitable for formulating such solid compositions may be used, e.g., magnesium stearate, starch, lactose, glucose, rice, flour and chalk. The compound may also be in the form of an ingestible capsule, e.g., of gelatin, to contain the compound, or in the form of a syrup, a solution or a suspension. Suitable liquid pharmaceutical carriers include ethyl alcohol, glycerine,

saline and water to which flavouring or colouring agents may be added to form syrups. Sustained release formulations, e.g., tablets containing an enteric coating, are also envisaged.

For parenteral administration, fluid unit dose forms are prepared containing the compound and a sterile vehicle. The compound depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants, such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

Preferred parenteral formulations include aqueous formulations using sterile water or normal saline, at a pH of around 7.4 or greater, in particular, containing penciclovir sodium salt hydrate.

As is common practice, the compositions will usually be accompanied by written or printed directions for use in the medical treatment concerned.

A suitable dosage unit might contain from 50-2,000 mg of active ingredient, e.g., 250-1,000 mg. Such doses may be administered as a one-day treatment regimen, such as 250 mg four times in one day, 1,500 mg once a day or 1,000 mg twice in one day.

The one-day treatment regimen of the present invention is preferably carried out as soon as possible after symptoms/signs appear usually within 48 hours, preferably 12 hours and more preferably within six hours of prodrome and/or of recurrence of genital herpes lesions. The treatment is particularly effective in the case of patients who are immunocompetent or experience multiple episodes of genital herpes per year but may also be suitable for patients who are not immunocompetent or do not have recurrent genital herpes.

The treatment period is one day.

Various types of lesions occur in genital herpes. Lesions requiring re-epithelialization for healing are those lesions which undergo vesicle, ulcer/soft crust and/or hard crust formation. Aborted lesions are lesions that develop no further than the papule stage. Prodrome alone may be considered the sign of an aborted lesion as utilized in the studies below. For purposes of the studies herein, if determination cannot be made due to missed visits or loss-of-follow-up, the patient will be assumed to have had lesions requiring re-epitheliatization in the unobserved period.

The stages of infection in genital herpes lesions which may be studied include presentation of papules, vesicle/pustules, ulcer/soft crust or hard crust. Papules refer to any swelling or solid superficial elevation of skin without fluid. Vesicle/Pustules refers to any presence of a blister-like elevation of the skin containing clear (vesicle) or cloudy (pustule) fluid, tiny red bumps or rash. A vesicle will un-roof in hours to days to evolve into an ulcer. Ulcer/soft crust refers to the collapse or rupture of a blister forming an ulcer. The floor of the ulcer may be moist. Ulcer may appear without evolving from vesicle. Hard crusting refers to the drying of the ulcer continued to form a noticeably hard, consolidated, firm mass, or a scab, an eschar. This marks the beginning of healing process. The virus may possibly still be present until the ulcer has completely healed, and the scab falls off.

Symptoms associated with the stages of genital herpes lesions include tenderness, pain, itching, burning and tingling.

The present invention also provides the use of a compound of formula (A) or a bioprecursor, or a pharmaceutically acceptable salt, phosphate ester and/or acyl derivative of either of the foregoing, in the preparation of a medicament for use in the treatment of recurrent herpes and in particular in reducing the duration of genital herpes lesions. Such treatment may be carried out in the manner as hereinbefore described.

The present invention further provides a pharmaceutical composition for use in the treatment of recurrent genital herpes, and in particular in reducing the duration of genital herpes lesions, which comprises an effective amount of a compound of formula (A) or a bioprecursor, or a pharmaceutically acceptable salt, phosphate ester and/or acyl derivative of either of the foregoing, and a pharmaceutically acceptable carrier. Such compositions may be prepared in the manner as hereinafter described.

The compound of formula (A) and its prodrugs show a synergistic antiviral effect in conjunction with interferons; and treatment using combination products comprising these two components for sequential or concomitant administration, by the same or different routes, are therefore within the ambit of the present invention. Such products are described in EP-A-271270 (Beecham Group p.l.c.).

The following clinical data illustrate the invention.

Clinical data

The study design incorporates patient-initiated therapy, which is consistent with the national guideline recommendation that herpes episodic treatment should be initiated as soon as possible after onset of symptoms [CDC (MMWR2002); and Drake 2000]. It is suggested that to ensure maximum benefit of episodic antiviral therapy, patients are advised to carry their medication with them and to start the treatment course as soon as a recurrence is suspected (Raj Patel, The Journal of Infectious Disease).

Design

The multi-center, multi-country, randomized, double-blind parallel-group, placebo controlled study is conducted to assess the efficacy and safety of a patient-initiated one-day treatment with famciclovir (Famvir) 1,000 mg, twice a day, in patients with recurrent genital herpes.

Approximately 450 eligible patients are randomized in a 1:1 ratio to receive either famciclovir (1,000 mg, twice a day) or matching placebo for one day. Patients are instructed to obtain a viral sample and initiate treatment within six hours of prodrome and/or recurrence of genital herpes lesions. All patients are required to return for clinic visits for observation of lesions on Days 2 and 3. Patients with lesions will continue with clinic visits on Days 4 and 5, then every other day until it is deemed that lesion healing has occurred however not to exceed Day 14. The following Table 1 represents the study design.

**Table1. Study Design**

| Pre-treatment Period | Double-blind period | | | | | | | |
|---|---|---|---|---|---|---|---|---|
| Screening | Day 0 | Day 1 | Day 2 | Day 3 | Day 4 | Day 5 | EOD | Study End |
| Randomization* | | | | | | | | |
| Visit 1 | Patient initiated therapy** | Visit 2*** | Visit 3 | Visit 4 | Visit 5 | Visit 6 | F/U† | Final‡ |
| Study Drug dispensed | Study Drug taken | Clinical follow-up | | | | | | |

* Double-blind 1-Day treatment study drug is dispensed to eligible patients
"With onset of prodrome and/or genital herpes lesion patient obtains viral sample and initiates within 6 hours treatment with study drug followed by a clinic visit within 24 hours of study treatment initiation
***If the first dose is taken PM on Day 0, then the second dose is to be taken AM on Day 1.
† Patients with lesions to be followed with clinic visit every other day (EOD) until complete re-epithelialization of the lesion occurs (erythema may be present)
‡ Final visit not to exceed Day 14

Each patient receives either Famciclovir 2,000 mg [1,000 mg (4 x 250 mg capsules)] orally or placebo (4 x 250 mg matching placebo capsules) orally twice in one day. Study medication is to be administered twice in one day (during normal waking hours). One-day equals to 24 hours, if the patent starts a first dose in the PM the second should be taken upon waking in the AM. The first dose is taken within six hours of prodrome and/or genital herpes lesion occurrence.

Measurements

Within 6 hours of recurrence of either genital herpes symptom onset and/or lesion and prior to initiating therapy, patients obtain a viral sample for PCR at Visit 1. The sample is brought to the clinic at Visit 2. Patients maintain a diary to assess and record appearance/stage and location of lesion. From Visit 2 onwards, physicals are performed by clinician who examines genitalia for herpes lesions. Patients with lesions will continue with clinic visit on Days 4 (Visit 5) and 5 (Visit 6) then every other day until clinician investigator deems that the lesion healing has occurred however not to exceed Day 14. Should patients only experience symptoms, without onset of herpes lesion, these patients will return for clinic visits until resolution of symptoms.

In addition to eligibility requirements, accurate diagnosis of genital herpes using laboratory confirmation is obtained. In one aspect, the type-specific serologic test that is based on glycoprotein assay is suitable to differentiate HSV-1 from HSV-2 infections in patients lacking laboratory documentation of being HSV2 positive [Tetrault 2000; and CDC (MMWR2002)].

In another aspect, PCR is selected over the standard culture sample to confirm the presence of viral DNA at genital sites. The data obtained provides a laboratory confirmation of symptomatic viral shedding which is another way to measure recurrent episodes.

Statistical analysis

All statistical tests are two-sided using $\alpha$=0.05.

Primary efficacy variable

The primary efficacy variable refers to the investigator-assessed time to healing (re-epithelization, erythema may be present) of all non-aborted genital herpes lesions from the time of the first dose of study medication. Non-aborted lesions refer to all lesions requiring re-epithelization: lesions which undergo vesicuar, ulcer/soft crust, hard crust formation. Aborted lesions refer to lesions that did not evolve beyond the papular stage and are assigned a time to healing of zero.

Primary efficacy variable is analyzed using the proportional hazards model, with treatment, gender and study center as explanatory variables, for patients over the modified ITT population. The Efron approximate likelihood method is used for resolution of ties. The 95% confidence intervals for the hazard ratio and median times to healing is calculated.

The distribution of time to healing of non-aborted genital herpes lesions are estimated using the Kaplan-Meier method and plotted for the modified ITT population, by gender, and by duration of genital herpes infection (dichotomized).

Sensitivity and exploratory analyses

The primary exploratory analysis is repeated over the per-protocol population for sensitivity purposes. The assumption of proportional hazards is assessed separately for treatment and gender using log-log survival plots. If marked non-proportionality is found for gender, the primary efficacy of variable is reanalyzed using gender as a stratifying factor. Effects of treatment regimens of various durations have been consistently shown in the literature as approximately proportional. However, if marked non-proportionality is found, such as when the treatment effect is larger in the first 2-3 days and smaller thereafter, presentation of the data by partitioning the time axis is considered. Covariates and interactions are assessed by adding their respective terms separately into the primary efficacy analysis models.

Secondary efficacy variables

Secondary efficacy variables refer to the investigator-assessed time to healing as in the primary efficacy variable and also include: time to confirmed resolution of all symptoms from the first dose of study medication, percentages of patients with pain, tenderness, itching, burning, and tingling, times to confirmed resolution of pain, tenderness, itching, burning, and tingling and the proportion of aborted lesions.

Analyses of secondary efficacy variables is performed on the nominal $\alpha$-level of 0.05, without adjusting for multiplicity.

The time to healing based on investigator-assessment (with aborted lesions assigned a time of zero) is analyzed on the PCR-positive and the ITT populations. A proportional hazards model is used in analysis, with treatment, gender, and study center as explanatory variables. The Efron approximate likelihood method is used for resolution of ties. A 95% confidence interval for the hazard ratio and median times to healing is calculated. Covariates and interactions listed for the primary efficacy variable are assessed separately by adding their terms into the proportional hazards model.

The time to resolution of each symptom, as well as the time to resolution of all symptoms, will be analyzed using a proportional hazards model with treatment, gender, and study center as explanatory variables, over the ITT population. The proportion of patients reporting each symptom is presented descriptively, and compared between treatments using the Chochran-Mantel-Haenszel 9CHM) test, stratified by gender and study center, over the ITT population. The proportion of aborted lesions will be presented descriptively, and compared between treatments using the CMH test, stratified by gender and study center, over the ITT and the PCR-positive populations.

The table below summarizes the results from Primary and Secondary efficacy analysis of famciclovir.

**Table 2. Primary and Secondary Efficacy Results**

| | Famciclovir 1,000 mg b.i.d. | Placebo | Hazard ratio | p-value |
|---|---|---|---|---|
| **Time to healing of non-aborted lesions (days)** | | | | |
| Modified ITT | 4.3<br>(3.9-5.0) | 6.1<br>5.0-7.0) | 1.64 | <0.001 |
| Per Protocol | 4.3<br>(3.9-5.5) | 6.2<br>(5.3-7.4) | 1.72 | <0.009 |
| **Time to healing of all lesions (days)[1]** | | | | |

(continued)

| | Famciclovir 1,000 mg b.i.d. | Placebo | Hazard ratio | p-value |
|---|---|---|---|---|
| ITT | 4.3 (3.9-5.0) | 4.3 (3.9-5.0) | | |
| **Proportion of aborted lesions** | | | | |
| ITT | 23.3% | 12.7% | | 0.003 |

1. Lesions that did not evolve beyond the papular stage were considered as aborted lesions and were assigned a time to healing of zero. The time to healing of an aborted lesion was not included in the determination of the primary endpoint.

Safety evaluation

The assessment of safety is based on reporting of adverse events. Adverse events are summarized by presenting for each treatment group, the number and percentage of patients having any adverse events, having an adverse event in each body system and having each individual adverse event. Any other information collected (e.g., severity of relatedness to study medication) is listed as appropriate.

Sample size and power considerations

The sample size of the study is based on the log-rank test on the time to lesion healing. In previous trials of famciclovir on this indication, the hazard ratio between famciclovir and placebo for time to healing has been estimated between 1.67 and 1.79 except in one case, with clinic-initiated treatment on a low dose (125 mg twice a day for five days), the estimated value was 1.48. As patient-initiated treatment is expected to provide more consistent results, the value of 1.47 is chosen as the smallest clinically meaningful hazard ratio. A combined total of 292 lesion healings is needed for 90% power in detecting such a difference between the treatment groups with type-one error rate of 5%. After accounting for approximately 35% of patients who do not develop lesions before the end of the study, or who drop out before confirmed healing is observed, the sample size of 225 patients needs to be randomized into each treatment group. To put the hazard ratio of 1.47 into the perspective of median time to healing, further distributional assumptions are made. The power of the statistical test is determined by the true hazard ratio and not affected by these distributional assumptions.

Based on data in the literature, the healing process of a patient population with recurrent genital herpes episodes tend to exhibit acceleration that cannot be characterized by the commonly-used exponential distribution, and may be better approximated by a Weibull distribution with survival function of the form

$$S(t)=\exp(-\lambda t^{\gamma})$$

By examining the quartiles in previous studies, a shape parameter of Y between 2 and 3 provides reasonable approximation. The between-treatment differences in median healing time under the above assumptions, with hazard ratio of 1.47 and placebo median between 4.5 and 6.5 days, are summarized in the table below.

Conclusion

The data illustrates famciclovir is superior to placebo in reducing the time to healing of nonaborted lesions (median time, 4.3 vs 6.1 days; $P<.001$) and all lesions (median time, 3.5 vs 5.0 days; $P<.001$). The proportion of patients with aborted lesions is significantly larger in the famciclovir group than in the placebo group (23.3% vs 12.7%; $P=.003$). Adverse events in the famciclovir group are infrequent overall, of mild to moderate severity, and similar to those in the placebo group.

Bioavailability of famciclovir is 77% regardless of the dose given (Pue 1993). Famciclovir 125 mg twice a day for a five-day treatment has been licensed for episodic genital herpes since 1995. Since then, more than four million patients participated in clinical trials worldwide without any concerns regarding famciclovir's safety. The safety of famciclovir at doses up to 1,500 mg per day (either 1,500 mg qd or 500 mg t.i.d.) for up to one year has been demonstrated in studies enrolling more than 700 patients (Allen et al. 1999, Trépo et al. 2000, De Man et al. 2000, Belgrave et al. 1999

and Beauchamp et al. 1999). The most commonly reported adverse events in patients receiving are headache and nausea.

If famciclovir is taken within six hours of the onset of symptoms of the aura that precedes a clinical lesion, a one-day, two-dose treatment of famciclovir reduces the median time to healing of recurrent genital herpes lesion as compared to placebo. These results are similar to those of registration studies with longer treatment duration (both Famvir and competitors). This study demonstrates that a one-day treatment with famciclovir is an effective, well-tolerated, and safe therapeutic alternative in reducing the time to healing of recurrent genital herpes lesions.

Famciclovir treatment is most effective when the patient has a clearly defined prodrome and initiates treatment promptly. Patient-initiated therapy, because it occurs earlier, has shown to be more effective than physician-initiated therapy for recurrent lesions. Use of famciclovir aborts viral shedding, accelerates lesion healing and relieves genital herpes symptoms in recurrent genital herpes.

## Claims

1. A method for the treatment of recurrent genital herpes in a human in need thereof, which method comprises administering to said human, an effective amount of the compound 9-(4-acetoxy-3-acetoxymethylbut-1-yl)-2-aminopurine (famciclovir), or a pharmaceutically acceptable salt thereof for a treatment period of one day.

2. A method according to Claim 1, wherein the one day treatment is commenced within 48 hours of rash onset.

3. A method according to Claim 2, wherein treatment is commenced within six hours of rash onset.

4. A method according to Claim 1, wherein famciclovir is administered at a dose of 2,000 mg.

5. A method according to Claim 1, wherein famciclovir is administered at a dose of 1,000 mg twice in one day.

6. A method according to Claim 1, wherein famciclovir is administered at a dose of 500 mg four times in one day.

7. A method according to Claim 1, wherein famciclovir is administered at a dose of 250 mg eight times in one day.

8. A method according to Claim 1, wherein famciclovir is administered orally.

9. A method according to Claim 1, wherein the compound is the sodium salt of penciclovir.

10. A method according to Claim 1, wherein the compound is administered parenterally.

Europäisches Patentamt
European Patent Office
Office européen des brevets

# EUROPEAN SEARCH REPORT

Application Number
EP 10 17 8109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHELLEY W B ET AL: ""Stat." single dose of acyclovir for prevention of herpes simplex.", CUTIS; CUTANEOUS MEDICINE FOR THE PRACTITIONER. JUN 1996, vol. 57, no. 6, June 1996 (1996-06), page 453, XP009071582, ISSN: 0011-4162 * the whole document * ----- | 1-10 | INV. A61K31/52 A61K31/522 A61P31/22 |
| A | US 5 840 763 A (FIELD ET AL) 24 November 1998 (1998-11-24) * column 3, line 23 - line 24 * * column 5, line 16 - line 36 * * claim 3 * ----- | 1-10 | |
| A | SACKS STEPHEN L ET AL: "A multicenter phase I/II dose escalation study of single-dose cidofovir gel for treatment of recurrent genital herpes", ANTIMICROBIAL AGENTS AND CHEMOTHERAPY, vol. 42, no. 11, November 1998 (1998-11), pages 2996-2999, XP002396614, ISSN: 0066-4804 * the whole document * ----- | 1-10 | TECHNICAL FIELDS SEARCHED (IPC) A61K |
| A | YANG H ET AL: "PROLONGED AND POTENT THERAPEUTIC AND PROPHYLACTIC EFFECTS OF S-1-3 HYDROXY-2-PHOSPHONYLMETHOXYPROPYL-CYTOSINE AGAINST HERPES SIMPLEX VIRUS TYPE 2 INFECTIONS IN MICE", ANTIMICROBIAL AGENTS AND CHEMOTHERAPY, vol. 35, no. 8, 1991, pages 1596-1600, XP002396615, ISSN: 0066-4804 * the whole document * ----- -/-- | 1-10 | |

The present search report has been drawn up for all claims

1

| Place of search | Date of completion of the search | Examiner |
|---|---|---|
| Munich | 3 November 2011 | Albrecht, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 17 8109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOYD M R ET AL: "ANTIHERPES VIRUS ACTIVITY OF 9-(4-HYDROXY-3-HYDROXYMETHYLBUT-1-YL) GUANINE (BRL 39123) IN ANIMALS", ANTIMICROBIAL AGENTS AND CHEMOTHERAPY, AMERICAN SOCIETY FOR MICROBIOLOGY, WASHINGTON, DC, US, vol. 32, no. 3, 1 March 1988 (1988-03-01), pages 358-363, XP000567471, ISSN: 0066-4804<br>* the whole document *<br>----- | 1-10 | |
| Y | SACKS S L ET AL: "Patient-initiated treatment (Tx) of recurrent genital herpes (RGH) with oral famciclovir (FCV): A Canadian, multicenter, placebo (PLB)-controlled, dose ranging study", ABSTRACTS OF THE INTERSCIENCE CONFERENCE ON ANTIMICROBIAL AGENTS AND CHEMOTHERAPY, vol. 34, no. 0, 1994, page 11, XP001247458, & 34TH INTERSCIENCE CONFERENCE ON ANTIMICROBIAL AGENTS AND CHEMOTHERAPY; ORLANDO, FLORIDA, USA; OCTOBER 4-7, 1994<br>* the whole document *<br>----- | 1-10 | TECHNICAL FIELDS SEARCHED (IPC) |

The present search report has been drawn up for all claims

1

| Place of search | Date of completion of the search | Examiner |
|---|---|---|
| Munich | 3 November 2011 | Albrecht, Silke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 8109

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2011

| Patent document cited in search report | Publication date | Patent family member(s) | Publication date |
|---|---|---|---|
| US 5840763 A | 24-11-1998 | AP 669 A | 04-09-1998 |
| | | AT 208198 T | 15-11-2001 |
| | | AU 699627 B2 | 10-12-1998 |
| | | AU 4257896 A | 03-07-1996 |
| | | BG 63815 B1 | 28-02-2003 |
| | | BG 101608 A | 27-02-1998 |
| | | BR 9509986 A | 25-11-1997 |
| | | CA 2207503 A1 | 20-06-1996 |
| | | CN 1175212 A | 04-03-1998 |
| | | CY 2339 B1 | 06-02-2004 |
| | | CZ 9701799 A3 | 17-09-1997 |
| | | DE 69523776 D1 | 13-12-2001 |
| | | DE 69523776 T2 | 01-08-2002 |
| | | DK 799039 T3 | 28-01-2002 |
| | | WO 9618396 A1 | 20-06-1996 |
| | | EP 0799039 A1 | 08-10-1997 |
| | | ES 2166834 T3 | 01-05-2002 |
| | | HK 1003259 A1 | 06-09-2002 |
| | | HU 222835 B1 | 28-11-2003 |
| | | JP 2008088189 A | 17-04-2008 |
| | | NO 972688 A | 07-08-1997 |
| | | NZ 297424 A | 28-07-2000 |
| | | PT 799039 E | 29-04-2002 |
| | | RO 120042 B1 | 30-08-2005 |
| | | SK 75197 A3 | 05-11-1997 |
| | | US 5916893 A | 29-06-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 141927 A
- EP 216459 A
- EP 182024 A
- EP 271270 A

### Non-patent literature cited in the description

- **BOYD.** *Abstracts of 14th Int. Congress of Microbiology,* 07 September 1986, 193